(19) Europäisches Patentamt
European Patent Office
Office européen des brevets

(11) EP 1 539 764 B1

(12) **EUROPEAN PATENT SPECIFICATION**

(45) Date of publication and mention of the grant of the patent:
**29.04.2009 Bulletin 2009/18**

(21) Application number: **03788213.1**

(22) Date of filing: **13.08.2003**

(51) Int Cl.:
***C07D 487/08*** *(2006.01)* ***A61K 31/551*** *(2006.01)*
***A61P 25/00*** *(2006.01)* ***A61P 1/04*** *(2006.01)*

(86) International application number:
**PCT/SE2003/001276**

(87) International publication number:
**WO 2004/016616 (26.02.2004 Gazette 2004/09)**

(54) **ARYL-SUBSTITUTED DIAZABICYCLOALKANES AS NICOTINIC ACETYLCHOLINE AGONISTS.**

ARYL-SUBSTITUIERTE DIAZABICYCLOALKANE ALS NIKOTINISCHE ACETYLCHOLINAGONISTEN

DIAZABICYCLOALCANES SUBSTITUES PAR ARYLE UTILISES COMME AGONISTES NICOTINIQUES DE L'ACETYLCHOLINE

(84) Designated Contracting States:
**AT BE BG CH CY CZ DE DK EE ES FI FR GB GR HU IE IT LI LU MC NL PT RO SE SI SK TR**

(30) Priority: **14.08.2002 SE 0202465**

(43) Date of publication of application:
**15.06.2005 Bulletin 2005/24**

(73) Proprietor: **AstraZeneca AB**
**151 85 Södertälje (SE)**

(72) Inventors:
- **ERNST, Glen,**
  **AstraZeneca Wilmington**
  **Wilmington, DE 19850-5437 (US)**
- **PHILLIPS, Eifion,**
  **AstraZeneca Wilmington**
  **Wilmington, DE 19850-5437 (US)**
- **SCHMIESING, Richard,**
  **AstraZeneca Wilmington**
  **Wilmington, DE 19850-5437 (US)**

(56) References cited:
**EP-A- 1 219 622** **WO-A-00/58311**

- **DATABASE CAPLUS [Online] DN 113:23956 XP002974068 Retrieved from STN Database accession no. 1990:423956 & US 4 895 943 A 23 January 1990**
- **DATABASE CAPLUS [Online] XP002974069 Retrieved from STN international, accession no. 1966:27623 Database accession no. 64:27623 & NL 65 001 367 A 04 August 1965**
- **DATABASE CAPLUS [Online] XP002974070 Retrieved from STN international, accession no. 1964:454808 Database accession no. 61:54808 & ZH. OBSHCH. KHIM. vol. 34, no. 7, 1964, pages 2222 - 6**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 539 764 B1

## Description

### Technical Field

This invention relates to diazabicycloalkane amides or pharmaceutically-acceptable salts thereof, processes for preparing them, pharmaceutical compositions containing them and their use in therapy. The invention also relates to compounds active as nicotinic acetylcholine receptors (nAChRs) agonists.

### Background of the Invention

The use of compounds which bind nicotinic acetylcholine receptors in the treatment of a range of disorders involving reduced cholinergic function such as Alzheimer's disease, cognitive or attention disorders, anxiety, depression, smoking cessation, neuroprotection, schizophrenia, analgesia, Tourette's syndrome, and Parkinson's disease has been discussed in McDonald et al. (1995) "Nicotinic Acetylcholine Receptors: Molecular Biology, Chemistry and Pharmacology", Chapter 5 in Annual Reports in Medicinal Chemistry, vol. 30, pp. 41-50, Academic Press Inc., San Dingo, CA; and in William et al. (1994) "Neuronal Nicotinic Acetylcholine Receptors," Drug News & Perspectives, vol. 7, pp. 205-223.

### Disclosure of the Invention

The invention comprises compounds of formula I

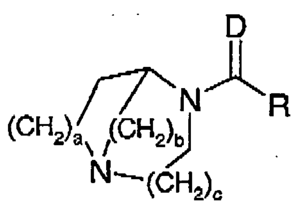

I

wherein:

a is 1, b is 2, and c is 1;
D is oxygen, and
R is selected from moieties of formulae II, III or IV:

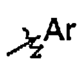

II

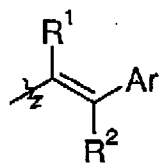

III

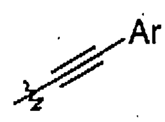

IV

wherein
$R^1$, and $R^2$ are independently selected from hydrogen, CN, $CF_3$, halogen, or $C_{1-4}$alkyl,;
Ar is phenyl, furanyl, thiophen-yl, naphthyl, benzofuranyl, benzo[b]thiophen-yl, or 1H indol;
wherein said Ar has 0, 1 or more substituents independently selected from $CF_3$, halogen, $C_{1-4}$akyl, or $OR^3$; where $R^3$ is $C_{1-4}$alkyl,, or

Another embodiment of the invention comprises compounds wherein Ar is a phenyl, furanyl or thiophenyl.
Particular compounds of the invention are those wherein a is 1, b is 2, c is 1, D is oxygen, $R^1$ and $R^2$ are hydrogen and Ar is phenyl, furanyl, thiophen-yl, naphthyl, benzofuranyl, benzo[b]thiophen-yl, or 1H-indol.
Particular compounds of the invention are also those wherein Ar is selected from phenyl, 2-furanyl or 3-furanyl, 2-thienyl or 3-thienyl, benzofuran-2-yl; benzofuran-3-yl, benzo[b]thiophen-2-yl or benzo[b]thiophen-3-yl.
Particular compounds of the invention are also those wherein Ar is substituted with one or more substituents

independently selected from $CF_3$, halogen, $C_{1-4}$alkyl, or $OR^3$.

Other particular compounds of the invention are:

(1,4-diazabicyclo[3.2.2]non-4-yl)(phenyl)methanone;
(1,4-diazabicyclo[3.2.2]non-4-yl)(3-fluorophenyl)methanone;
(1,4-diazabicyclo[3.2.2]non-4-yl)(4-fluorophenyl)methanone;
(3-chlorophenyl)(1,4-diazabicyclo[3.2.2]non-4-yl)methanone;
(4-chlorophenyl)(1,4-diazabicyclo[3.2.2]non-4-yl)methanone;
(1,4-diazabicyclo[3.2.2]non-4-yl)(3,4-dichlorophenyl)methanone;
(3-bromophenyl)(1,4-diazabicyclo[3.2.2]non-4-yl)methanone;
(4-bromophenyl)(1,4-diazabicyclo[3.2.2]non-4-yl)methanone;
(1,4-diazabicyclo[3.2.2]non-4-yl)(3-iodophenyl)methanone;
(1,4-diazabicyclo[3.2.2]non-4-yl)(4-iodophenyl)methanone;
(1,4-diazabicyclo[3.2.2]non-4-yl)(4-trifluoromethylphenyl)methanone;
(1,4-diazabicyclo[3.2.2]non-4-yl)(4-methoxyphenyl)methanone;
(1,4-diazabicyclo[3.2.2]non-4-yl)(4-trifluoromethoxyphenyl)methanone;
(5-chlorofuran-2-yl)(1,4-diazabicyclo[3.2.2]non-4-yl)methanone;
(5-bromofuran-2-yl)(1,4-diazabicyclo[3.2.2]non-4-yl)methanone;
(5-iodoofuran-2-yl)(1,4-diazabicyclo[3.2.2]non-4-yl)methanone;
(5-chlorothiophen-2-yl)(1,4-diazabicyclo[3.2.2]non-4-yl)methanone;
(5-bromothiophen-2-yl)(1,4-diazabicyclo[3.2.2]non-4-yl)methanone;
(5-iodoothiophen-2-yl)(1,4-diazabicyclo[3.2.2]non-4-yl)methanone;
(1,4-diazabicyclo[3.2.2]non-4-yl)(naphthalen-2-yl)methanone;
(1,4-diazabicyclo[3.2.2]non-4-yl)(benzofuran-2-yl)methanone;
(1,4-diazabicyclo[3.2.2]non-4-yl)(benzo[b]thiophen-2-yl)methanone;
1-(1,4-diazabicyclo[3.2.2]non-4-yl)-3-phenylpropenone;
1-(1,4-diazabicyclo[3.2.2]non-4-yl)-3-phenylpropynone;
1-(1,4-diazabicyclo[3.2.2]non-4-yl)-3-(furan-2-yl)propenone;
1-(1,4-diazabicyclo[3.2.2]non-4-yl)-3-(furan-3-yl)propenone;
1-(1,4-diazabicyclo[3.2.2]non-4-yl)-3-(thiophen-2-yl)propenone;
1-(1,4-diazabicyclo[3.2.2]non-4-yl)-3-(thiophen-3-yl)propenone;
(1,4-diazabicyclo[3.2.2]non-4-yl)(furan-2-yl)methanone;
(E)-1-(1,4-diazabicyclo[3.2.2]non-4-yl)-3-(furan-2-yl)propenone;
(E)-1-(1,4-diazabicyclo[3.2.2]non-4-yl)-3-(thiophen-2-yl)propenone;
(E)-1-(1,4-diazabicyclo[3.2.2]non-4-yl)-3-(2-methoxyphenyl)-propenone;
(E)-1-(1,4-diazabicyclo[3.2.2]non-4-yl)-3-(2-methylphenyl)propenone;
(1,4-diaza-bicyclo[3.2.2]non-4-yl)-(1H-indol-5-yl)-methanone;
(1,4-diaza-bicyclo[3.2.2]non-4-yl)-(methyl-1H-indol-2-yl)-methanone, and
(Z)-1-(1,4-diaza-bicyclo[3.2.2]non-4-yl)-2-fluoro-3-phenyl-propenone.

Most particular compounds of the invention are those of the examples herein.

Each embodiment and particular form of the invention encompass all diastereoisomers, enantiomers and pharmaceutically-acceptable derivatives and salts of compounds thereof.

Pharmaceutically-acceptable derivatives include solvates and salts. For example, the compounds of formula I can form acid addition salts with acids, such as the conventional pharmaceutically-acceptable acids, for example, maleic, hydrochloric, hydrobromic, phosphoric, acetic, fumaric, salicylic, citric, lactic, mandelic, tartaric and methanesulfonic acids.

Compounds of the invention are useful in the treatment or prophylaxis of human diseases or conditions in which activation of the $\alpha 7$ nicotinic receptor is beneficial as well as in the treatment or prophylaxis of psychotic disorders or intellectual impairment disorders. Examples of such conditions, diseases or disorders are Alzheimers disease, learning deficit, cognition deficit, attention deficit, memory loss, Attention Deficit Hyperactivity Disorder, Anxiety, schizophrenia, mania or manic depression, Parkinson's disease, Huntington's disease, Tourette's syndrome, neurodegenerative disorders in which there is loss of cholinergic synapse, jetlag, cessation of smoking, nicotinic addiction including that resulting from exposure to products containing nicotine, pain, for ulcerative colitis and irritable bowel disease.

As used herein, unless otherwise indicated, "$C_{1-4}$alkyl" includes but is not limited to methyl, ethyl, n-propyl, n-butyl, i-propyl, i-butyl, t-butyl, s-butyl moieties, whether alone or part of another group, $C_{1-4}$alkyl groups may be straight-chained or branched, and $C_{3-4}$alkyl groups include the cyclic alkyl moieties cyclopropyl and cyclobutyl. Alkyl groups referred to herein may have 1, 2 or 3 halogen substituents.

As used herein, unless otherwise indicated, "$C_{2-4}$alkenyl" includes but is not limited to 1-propenyl, 2-propenyl, 1-butenyl, 2-butenyl and 3-butenyl.

As used herein, unless otherwise indicated, "$C_{2-4}$alkynyl" includes but is not limited to ethynyl, 1-propynyl, 2-propynyl, 1-butynyl, 2-butynyl and 3-butynyl.

As used herein, unless otherwise indicated, aryl refers to a phenyl ring which may have 1, 2 or 3 substituents selected from CN, $NO_2$, $CF_3$, halogen, $C_{1-4}$alkyl, $C_{2-4}$alkenyl, $C_{2-4}$alkynyl, $OC_{1-4}$alkyl, $NH_2$ and $CO_2C_{1-4}$alkyl.

As used herein, unless otherwise indicated, heteroaryl refers to a 5- or 6-membered aromatic or heteroaromatic ring having 0, 1, 2 or 3 nitrogen atoms, 0 or 1 oxygen atom, and 0 or 1 sulfur atom, provided that the ring contains at least one nitrogen, oxygen, or sulfur atom. Heteroaryl moieties may have one or more substituents selected from CN, $NO_2$, $CF_3$, halogen, $C_{1-4}$alkyl, $C_{2-4}$alkenyl, $C_{2-4}$alkynyl, $NH_2$, $CO_2H$, $OC_{1-4}$alkyl and $CO_2C_{1-4}$alkyl.

As used herein, unless otherwise indicated, halogen refers to fluorine, chlorine, bromine, or iodine.

The preparation of 1,4-diazabicyclo(3.2.2)nonane has been described in WO-A-00/58311.

## Methods of Preparation

In the reaction schemes and text that follow, D and R, unless otherwise indicated, are as defined above for formula I. The compounds of formula I may be prepared according to the methods outlined in Scheme 1.

**Scheme 1**

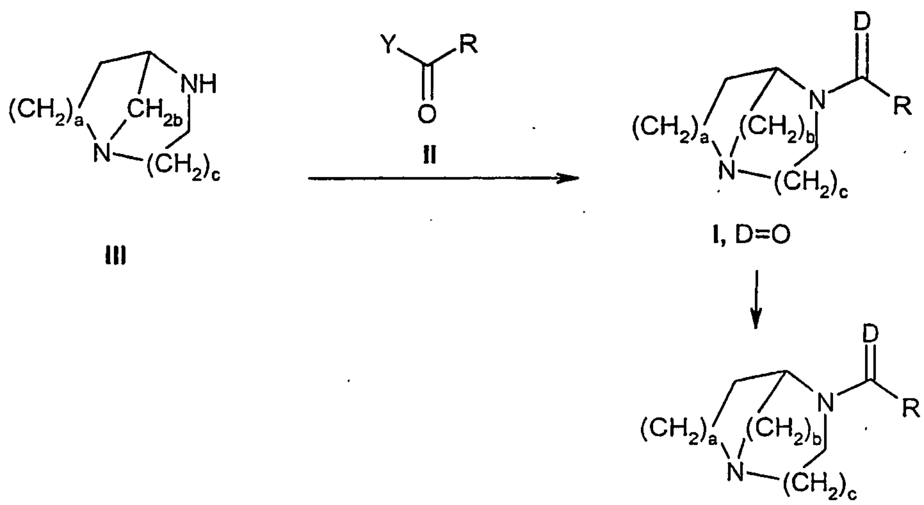

III

II

I, D=O

I, D=S

Compounds of formula I wherein D represents O may be prepared from compounds of formula III by reaction with a compound of formula II, wherein Y represents a suitable leaving group, using a suitable acylation procedure. Suitable leaving groups Y include: OH, halogen, Oalkyl, Oaryl, OCOalkyl, OCOaryl, azide. A suitable acylation procedure involves treatment of a compound of formula III with a compound of formula II at 0-120 ˚C in a suitable solvent. The presence of a base, or, when Y=OH, a coupling agent, may also be necessary for the reaction to occur. Suitable bases for the reaction include: 4-(*N*,*N*-dimethylamino)pyridine, pyridine, triethylamine, *N*,*N*-diisopropylethylamine. The preferred base is *N*,*N*-diisopropylethylamine. Suitable coupling agents when Y=OH include: carbodiimides, for example 1,3-dicyclohexylcarbodiimide or 1-(3-dimethylaminopropyl-3-ethylcarbodiimide hydrochloride; phosphonium reagents, for example benzotriazol-1-yloxytris(dimethylamino)phosphonium hexafluorophosphate or benzotriazol-1-yloxytripyrrolidinophosphonium hexafluorophosphate; and uronium reagents, for example *O*-benzotriazol-1-yl-*N*,*N*,*N*',*N*'-tetramethyluronium tetrafluoroborate. The preferred coupling agent is *O*-benzotriazol-1-yl-*N*,*N*,*N*',*N*'-tetramethyluronium tetrafluoroborate. Suitable solvents for the reaction include *N*,*N*-dimethylformamide, dimethylsulfoxide, tetrahydrofuran, or chloroform. The preferred solvent is *N*,*N*-dimethylformamide. The reaction is preferably performed at a temperature of 0-50 ˚C, and most preferably at a temperature of 20-30 ˚C.

Compounds of formula I in which D represents S may be prepared from compounds of formula I in which D

represents O by reaction with a suitable sulfide in a suitable solvent.

The preferred sulfides are phosphorus sulfides, in particular 4-methoxyphenylthionophosphine sulfide dimer ("Lawesson's Reagent"), and diphosphorus pentasulfide. Suitable solvents for the reaction include aryl hydrocarbon solvents, for example toluene or xylene. The reaction is performed at a temperature of 0-200 ˚C, and preferably at a temperature of 50-180˚C.

It will be appreciated by one skilled in the art that certain optional aromatic substituents in the compounds of the invention may be introduced by employing aromatic substitution reactions, or functional group transformations to modify an existing substituent, or a combination thereof. Such reactions may be effected either prior to or immediately following the processes mentioned above, and are included as part of the process aspect of the invention. The reagents and reaction conditions for such procedures are known in the art. Specific examples of procedures which may be employed include, but are not limited to, electrophilic functionalisation of an aromatic ring, for example via nitration, halogenation, or acylation; transformation of a nitro group to an amino group, for example via reduction, such as by catalytic hydrogenation; acylation, alkylation or sulfonylation of an amino or hydroxyl group; replacement of an amino group by another functional group via conversion to an intermediate diazonium salt followed by nucleophilic or free radical substitution of the diazonium salt; or replacement of a halogen by another functional group for example via nucleophilic or catalysed substitution reactions.

Where necessary, hydroxy, amino, or other reactive groups may be protected using a protecting group as.described in the standard text "Protecting groups in Organic Synthesis", 3rd Edition (1999) by Greene and Wuts.

The above described reactions, unless otherwise noted, are usually conducted at a pressure of about one to about three atmospheres, preferably at ambient pressure (about one atmosphere). Unless otherwise stated, the above described reactions are conducted under an inert atmosphere, preferably under a nitrogen atmosphere.

The compounds of the invention and intermediates may be isolated from their reaction mixtures by standard techniques.

Acid addition salts of the compounds of formula I which may be mentioned include salts of mineral acids, for example the hydrochloride and hydrobromide salts; and salts formed with organic acids such as formate, acetate, maleate, benzoate, tartrate, and fumarate salts.

Acid addition salts of compounds of formula I may be formed by reacting the free base or a salt, enantiomer or protected derivative thereof, with one or more equivalents of the appropriate acid. The reaction may be carried out in a solvent or medium in which the salt is insoluble or in a solvent in which the salt is soluble, e.g., water, dioxane, ethanol, tetrahydrofuran or diethyl ether, or a mixture of solvents, which may be removed in vacuum or by freeze drying. The reaction may be a metathetical process or it may be carried out on an ion exchange resin.

The compounds of formula I exist in tautomeric or enantiomeric forms, all of which are included within the scope of the invention. The various optical isomers may be isolated by separation of a racemic mixture of the compounds using conventional techniques, e.g. fractional crystallization, or chiral HPLC. Alternatively the individual enantiomers may be made by reaction of the appropriate optically active starting materials under reaction conditions which will not cause racemization,

<u>Example 1:</u> **(1,4-Diazabicyclo[3.2.2]non-4-yl)(phenyl)methanone**

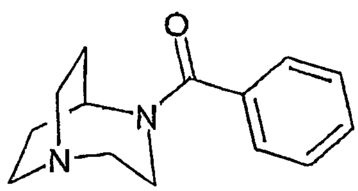

Benzoic acid (61 mg, 0.50 mmol), 1,4-diaza-bicyolo[3.2.2]nonane dihydrochloride (100 mg, 0.50 mmol), 1-hydroxybenzotriazole hydrate (68 mg, 0.50 mmol), O-(benzotriazol-1-yl)-N,N,N',N'-tetramethyluronium tetrafluoroborate (161 mg, 0.50 mL) and diisopropylethylamine (0.35 mL, 250 mg, 2,0 mmol) in dry N,N-dimethylformamide (2 mL) were stirred at ambient temperature for 89 h. The reaction mixture was poured into 1N sodium hydroxide solution and extracted with ethyl acetate. The ethyl acetate layer was washed with 1N NaOH (1x), water (4x), brine (1x), and dried over $MgSO_4$. After filtration, the solvent was removed *in vacuo* to yield (1,4-diaza-bicyclo[3.2.2]non-4-yl)(phenyl)methanone (13 mg, 11%) as a tan waxy solid.
MS (APCI+) 231 $[M+1]^+$.

**<u>Example 2:</u> (4-Chlorophenyl)(1,4-diazabicyclo[3.2.2]non-4-yl)methanone**

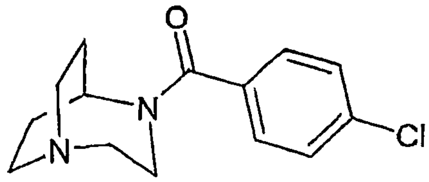

4-Chlorobenzoic acid (79 mg, 0.50 mmol), 1,4-diaza-bicyclo[3.2.2]nonane dihydrochloride (100 mg, 0.50 mmol), 1-hydroxybenzotriazole hydrate (68 mg, 0.50 mmol), O-(benzotriazol-1-yl)-N,N,N',N'-tetramethyluronium tetrafluoroborate (161 mg, 0.50 mL) and diisopropylethylamine (0.35 mL, 250 mg, 2.0 mmol) in dry N,N-dimethylformamide (2 mL) were stirred at ambient temperature for 89 h. The reaction mixture was poured into 1N sodium hydroxide solution and extracted with ethyl acetate. The ethyl acetate layer was washed with 1N NaOH (1x), water (4x), brine (1x), and dried over $MgSO_4$. After filtration, the solvent was removed *in vacuo* to yield (4-chlorophenyl)(1,4-diazabicyclo[3.2.2]non-4-yl)methanone (73 mg, 55%) as a tan oil.
MS (APCI+) 265/267 [M+1]+; $^1$H-NMR (300 MHz, $CDCl_3$): δ 7.49 (2H, d), 7,40 (2H, d), 4.58-4,50 (1H, m), 3.83-3.68 (1H, m), 3.48-3.36 (1H, m), 3.02-2.75 (6H, m), 2.08-1.45 (4H, m).

**<u>Example 3:</u> (1,4-Diazabicyclo[3.2.2]non4-yl)(4-methoxyphenyl)methanone**

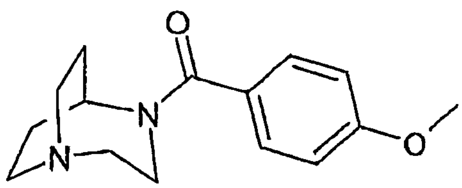

4-Methoxybenzoic acid (76 mg, 0.50 mmol), 1,4-diaza-bicyclo[3.2.2]nonane dihydrochloride (100 mg, 0.50 mmol), 1-hydroxybenzotriazole hydrate (68 mg, 0.50 mmol), O-(benzotriazol-1-yl)-N,N,N',N'-tetramethyluronium tetrafluoroborate (161 mg, 0.50 mL) and diisopropylethylamine (0.35 mL, 250 mg, 2.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (1,4-diazabicyclo[3.2.2]non-4-yl)(4-methoxyphenyl)methanone (13 mg, 10%) as a colorless resin.
MS (APCI+) 261 [M+1]+; $^1$H-NMR (300 MHz, $CDCl_3$): δ 7.33 (2H, d), 6.96 (2H, d), 4,62-4.40 (1H, m), 3.80 (2H, br s), 3.78 (3H, s), 2.99-2.76 (6H, m), 2.09-1.47 (4H, m).

**<u>Example 4:</u> (1,4-Diazabicyclo[3.2.2]non-4-yl)(benzofuran-2-yl)methanone**

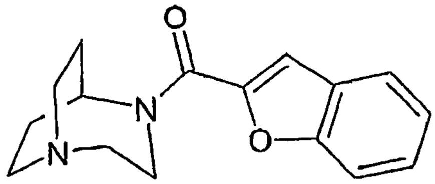

Benzofuran-2-carhoxylic acid (81 mg, 0.50 mmol), 1,4-diaza-bicyclo[3.2.2]nonane dihydrochloride (100 mg, 0.50 mmol), 1-hydroxybenzotriazole hydrate (68 mg, 0.50 mmol), O-(benzotriazol-1-yl)-N,N,N',N'-tetramethyluronium tetrafluoroborate (161 mg, 0.50 mL) and diisopropylethylamine (0.35 mL, 250 mg, 2.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (1,4-diazabicyclo[3.2.2]non-4-yl)(benzofuran-2-yl)methanone (46 mg, 34%) as a yellow solid.
MS (APCI+) 271 [M+1]+; $^1$H-NMR (300 MHz, $CDCl_3$): δ 7.74 (1H, d), 7.65 (1H, d), 7.43 (1H, dd), 7.38-7.28 (2H, m), 4.59-4.38 (1H, m), 3.91-3.73 (2H, m), 3.00-2.85 (6H, m), 2.09-1.91 (2H, m), 1.83-1,64 (2H, m).

<u>Example 5:</u> **(E)-1-(1,4-Diazabicyclo[3.2.2]non-4-yl)-3-(furan-2-yl)propenone**

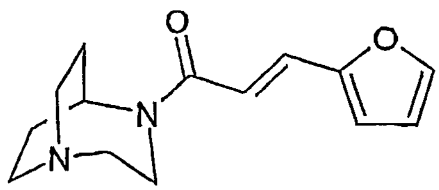

(E)-3-Furan-2-yl-acrylic acid (69 mg, 0.50 mmol), 1,4-diaza-bicyclo[3,2.2]nonane dihydrochloride (100 mg, 0.50 mmol), 1-hydroxybenzotriazole hydrate (68 mg, 0.50 mmol), O-(benzotriazol-1-yl)-N,N,N',N'-tetramethyluronium tetrafluoroborate (161 mg, 0.50 mL) and diisopropylethylamine (0.35 mL, 250 mg, 2.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (E)-1-(1,4-diazabicyclo[3.2.2]non-4-yl)-3-(furan-2-yl)propenone (49 mg, 40%) as a beige solid,
MS (APCI+) 247 [M+1]+ $^1$H-NMR (300 MHz, $CDCl_3$): δ 7.98-7.73 (1H, m), 7.42-7.23 (1H, m), 6.97-6.76 (2H, m), 6.63-6.53 (1H, m), 4.56-4.26 (1H, m), 3.80-3.66 (2H, m), 3.02-2.77 (6H, m), 1.97-1.53 (4H, m),

<u>Example 6:</u> **(E)-1-(1,4-Diazabicyclo[3.2.2]non-4-yl)-3-(thiophen-2-yl)propenone**

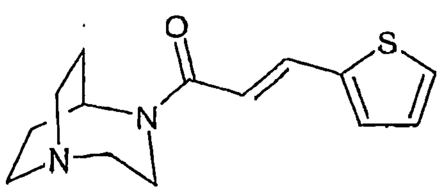

(E)-3-Thiophen-2-yl-acrylic acid (77 mg, 0.50 mmol), 1,4-diaza-bicyclo[3.2.2]nonane dihydrochloride (100 mg, 0.50 mmol), 1-hydroxybenzotriazole hydrate (68 mg, 0.50 mmol), O-(benzotriazol-1-yl)-N,N,N',N'-tetramethyluronium tetrafluoroborate (161 mg, 0.50 mL) and diisopropylethylamine (0.35 mL, 250 mg, 2.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (E)-1-(1,4-diazabicyclo[3.2.2]non-4-yl)-3-(thiophen-2-yl)propenone (62 mg, 47%) as a colorless oil.
MS (APCI+) 263 [M+1]+; $^1$H-NMR (300 MHz, $CDCl_3$): δ 7.73-7.55 (2H, m), 7.48-7.42 (1H, m), 7.15-7.01 (1H, m), 6.96-6.76 (1H, m), 4.56-4.31 (1H, m), 3,79-3.70 (2H, m), 2.99-2.77 (6H, m), 1,97-1.54 (4H, m).

**Example 7: (E)-1-(1,4-Diazabicyclo[3.2.2]non-4-yl)-3-(2-methoxyphenyl)-propenone**

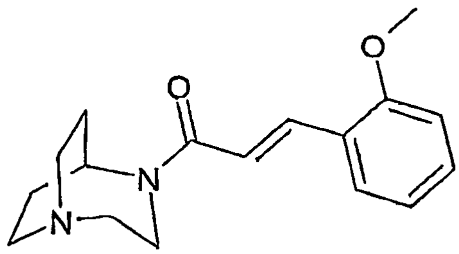

(E)-3-(2-Methoxyphenyl)acrylic acid (89 mg, 0.50 mmol), 1,4-diazabicyclo[3.2.2]nonane dihydrochloride (100 mg, 0.50 mmol), 1-hydroxybenzotriazole hydrate (68 mg, 0.50 mmol), O-(benzotriazol-1-yl)-N,N,N',N'-tetramethyluronium tetrafluoroborate (161 mg, 0.50 mL) and diisopropylethylamine (0.35 mL, 250 mg, 2.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (E)-1-(1,4-diazabicyclo[3.2.2]non-4-yl)-3-(2-methoxyphehyl)propenone (74 mg, 52%) as a yellow solid.
MS (APCI+) 287 [M+1]+; $^1$H-NMR (300 MHz, $CDCl_3$): δ 7.97-7.67 (2H, m), 7.41-7.30 (1H, m), 7.23-6.92 (3H, m), 4.57-4.35 (1H, m), 3.85 (3H, s), 3.81-3.72 (2H, m), 3.02-2.78 (6H, m), 1.97-1.54 (4H, m).

**Example 8: (E)-1-(1,4-Diazabicyclo[3.2.2]non4-yl)-3-(2-methylphenyl)propenone**

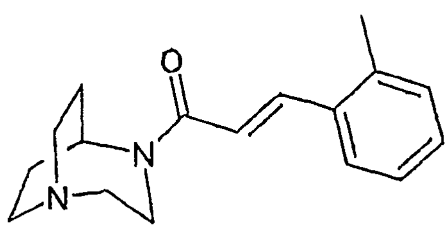

(E)-3-(2-Methylphenyl)acrylic acid (81 mg, 0.50 mmol), 1,4-diazabicyclo[3.2.2]nonane dihydrochloride (100 mg, 0.50 mmol), 1-hydroxybenzotriazole hydrate (68 mg, 0.50 mmol), O-(benzotriazol-1-yl)-N,N,N',N-tetramethyluronium tetrafluoroborate (161 mg, 0.50 mL) and diisopropylethylamine (0.35 mL, 250 mg, 2.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (E)-1-(1,4-diazabicyclo[3,2,2]non-4-yl)-3-(2-methylphenyl)propenone (76 mg, 56%) as a colorless oil.
MS (APCI+) 271 [M+I]+; $^1$H-NMR (300 MHz, $CDCl_3$): δ 7.83-7.64 (2H, m), 7.32-7.17 (3H, m), 7.16-6.96 (1H, m), 4.57-4.41 (1H, m), 3.83-3.72 (2H, m), 3.00-2.77 (6H, m), 2.37 (3H, s), 2.00-1.54 (4H, m).

**Example 9: (1,4-Diaza-bicyclo[3.2.2]non-4-yl)-(1H-indol-5-yl)-methanone**

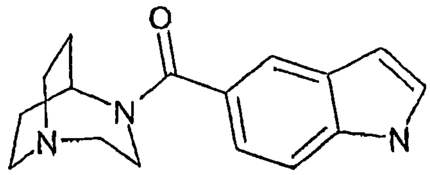

Indole-5-carboxylic acid (40 mg, 0.25 mmol), 1,4-diaza-bicyclo[3.2.2]nonane dihydrochloride (50 mg, 0.25 mmol), 1-hydroxybenzotriazole hydrate (34 mg, 0.25 mmol), O-(benzotriazol-1-yl)-N,N,N',N'-tetramethyluronium tetrafluoroborate (81 mg, 0.25 mmol) and diisopropylethylamine (0,17 mL, 129 mg, 1.0 mmol) in dry N,N-dimethylformamide (1.5 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield 10 mg of product. The reaction mixture was chromatographed with 100% EtOAc to 90:10 EtOAc:7N $NH_3$/MeOH to give (1,4-diazabicyclo[3.2.3]non-4-yl)-(1H-indol-5-yl)-methanone (5 mg, 7%) as a pale yellow oil.
MS (APCI+) 270 $[M+1]^+$; $^1$H-NMR (300 MHz, $CDCl_3$): δ 8.67 (1H, s), 7.68 (1H, s), 7.35 (1H, d), 7.26-7.20 (2H, m), 6.56 (1H, s), 4.81 (1H, s), 3.67-3.66 (2H, m), 3.07-2.97 (6H, m), 2,13-2.00 (2H, m), 1.77 (2H, s).

**<u>Example 10:</u> (1,4-Diaza-bicyclo[3.2.2]non-4-yl)-(naphthylene-2-yl)-methanone**

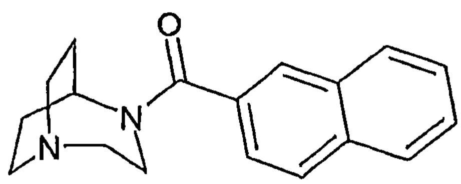

2-Napthoic acid (43 mg, 0.25 mmol), 1,4-diaza-bicyclo[3.2.2]nouane dihydrochloride (50 mg, 0.25 mmol), 1-hydroxybenzotriazole hydrate (34 mg, 0.25 mmol), O-(benzotriazol-1-yl)-N,N,N',N'-tetramethyluronium tetrafluoroborate (81 mg, 0.25 mmol) and diisopropylethylamine (0.17 mL, 129 mg, 1,0 mmol) in dry N,N-dimethylformamide (1.5 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield 50 mg of product. The reaction mixture was chromatographed with 100% EtOAc to 90:10 EtOAc:7N $NH_3$/MeOH to give (1,4-diazabicyclo[3.2.2]non-4-yl)-naphthalen-2-yl-methanone (46 mg, 66%) as a colorless oil.
MS (APCI+) 281 [M+1]+; $^1$H-NMR (300 MHz, $CDCl_3$): δ 7,89-7.84 (4H, m), 7.61-7.46 (3H, m), 4.84 (1H, s), 3.59 (1H, s), 3.15-2.94 (7H, m), 2.18 (2H, s), 1.83 (2H, s) 1.66 (1H, s).

**<u>Example 11:</u> (1,4-Diaza-bicyclo[3.2.2]non-4-yl)-(methyl-1H-indol-2-yl)-methanone**

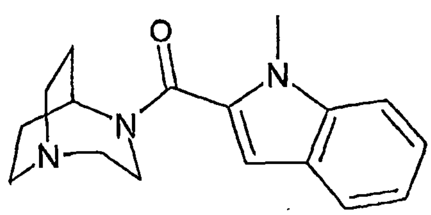

1-Methylindolc-2-carboxylic acid (44 mg, 0.25 mmol), 1,4-diazabicyclo[3.2.2]nonane dihydrochloride (50 mg,

0.25 mmol), 1-hydroxybenzotriazole hydrate (34 mg, 0.25 mmol), O-(benzotriazol-1-yl)-N,N,N',N'-tetramethyluronium tetrafluoroborate (81 mg, 0.25 mmol) and diisopropylethylamine (0.17 mL, 129 mg, 1.0 mmol) in dry N,N-dimethylformamide (1.5 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield 54 mg of product. The reaction mixture was chromatographed with 100% EtOAc to 90:10 EtOAc:7N $NH_3$/MeOH to give (1,4-diaza-bicyclo[3.2.2]non-4-yl)-(1-methyl-1H-indol-2-yl)-methanone (48 mg, 68%) as a colorless oil.
MS (APCI+) 284 [M+1]+; $^1$H-NMR (300 MHz, $CDCl_3$): δ 7.62 (1H, d), 7.39-7.26 (2H, m), 7.16 (1H, dd) 6.56 (1H, s), 4.80 (1H, s), 3.86-3.77 (5H, m), 3.07-3.02 (7H, m), 2.04 (2H, s), 1.81 (2H, s) 1.66 (1H, s).

**Example 12:** **(Z)-1-(1,4-Diaza-bicyclo[3.2.2]non-4-yl)-2-fluoro-3-phenyl-propenone**

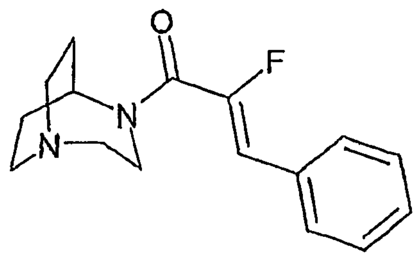

α-fluorocinnamic acid (42 mg, 0.25 mmol), 1,4-diaza-bicyclo[3.2.2]nonane dihydrochloride (50 mg, 0.25 mmol), 1-hydroxylbenzotriazolc hydrate (34 mg, 0.25 mmol), O-(benzotriazol-1-yl)-N,N,N',N'-tetramethyluronium tetrafluoroborate (81 mg, 0.25 mmol) and diisopropylethylamine (0.17 mL, 129 mg, 1.0 mmol) in dry N,N-dimethylformamide (1.5 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield 61 mg of product. The reaction mixture was chromatographed with 100% EtOAc to 90:10 EtOAc:7N $NH_3$/MeOH to give (Z)-1-(1,4-diaza-bicyclo[3.2.2]non-4-yl)-2-fluoro-3-phenyl-propenone (54 mg, 78%) as a colorless oil.
MS (APCI+) 275 [M+1]+; $^1$H-NMR (300 MHz, $CDCl_3$): δ 7.57 (2H, d), 7,40-7.29 (3H, m), 6.49 (1H, d), 4.62 (1H, s), 3.75 (2H, s), 3.15-2.95 (7H, m), 2.06-2.02 (2H, m), 1.79 (2H, s),

**Example 13:** **1-(1,4-Diaza-bicyclo[3.2.2]non-4-yl)-3-phenyl-propynone**

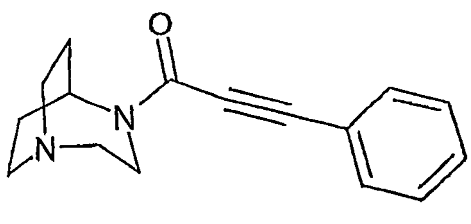

Phenylpropionic acid (37 mg, 0.25 mmol), 1,4-diaza-bicyclo[3,2,2]nonane dihydrochloride (50 mg, 0.25 mmol), 1-hydroxybenzotriazole hydrate (34 mg, 0.25 mmol), O-(benzotriazol-1-yl)-N,N,N',N'-tetramethyluronium tetrafluoroborate (81 mg, 0.25 mmol) and diisopropylethylamine (0.17 mL, 129 mg, 1.0 mmol) in dry N,N-dimethylformamide (1.5 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield 45 mg of product. The reaction mixture was chromatographed with 100% EtOAc to 90:10 EtOAc:7N $NH_3$/MeOH to give 1-(1,4-diazabicyclo[3.2.2]non-4-yl)-3-phenyl-propynone (38 mg, 59%) as a colorless oil,
MS (APCI+) 255 [M+1]+; $^1$H-NMR (300 MHz, $CDCl_3$): δ 7.61-7.51 (2H, m), 7,45-7.33 (3H, m), 4.68-4.62 (1H, m), 4,00

(1H, t), 3.86 (1H, t), 3.17-2.94 (6H, m), 2.12-1,99 (2H, m), 1.88-1.68 (3H, m).

**Example 13: (1,4-diazabicyclo[3.2.2]non-4-yl)(benzo[b]thiophen-2-yl)methanone dihydrochloride.**

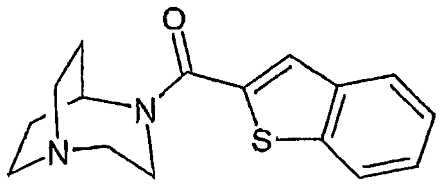

To a stirred mixture of 1,4-diazabicyclo[3.2.2]nonane dihydrochloride (100 mg, 0.51 mmol), triethylamine (0,3 mL) and a catalytic amount of N,N-dimethylaminopyridine in dry THF (2.5 mL) at ambient temperature was added a solution of benzo[b]thiophene-2-carbonyl chloride in dry THF (0.5 mL). After stirring at ambient temperature for 2 hours the mixture was partitioned between water and ethyl acetate, the organic phases recovered, washed with water and brine, then dried over sodium sulfate. The product obtained by concentration of the dried organic phases was subjected to silica gel chromatography, eluting with an ammoniated-chloroform to 5% methanol/chloroform gradient to give the title compound as a free base. The eluted material was dried to a solid. The solid was taken up in methanol, made acidic with HCl in ether (2.0 M), diluted with ether and allowed to stand. The resulting salt was collected, washed, and dried *in vacuo* to give the title compound as a white solid (55.0 mg). MS (ES+) 287 (MH+).

## Pharmaceutical compositions

A further aspect of the invention relates to a pharmaceutical composition for treating or preventing a condition or disorder as exemplified below arising from dysfunction of nicotinic acetylcholine receptor neurotransmission in a mammal, preferably a human, comprising an amount of a compound of formula I, an enantiomer thereof or a pharmaceutically acceptable salt thereof, effective in treating.or preventing such disorder or condition and an inert pharmaceutically acceptable carrier.

For the above-mentioned uses the dosage administered will, of course, vary with the compound employed, the mode of administration and the treatment desired. However, in general, satisfactory results are obtained when the compounds of the invention are administered at a daily dosage of from about 0.1 mg to about 20 mg per kg of animal body weight, preferably given in divided doses 1 to 4 times a day or in sustained release form. For man, the total daily dose is in the range of from 5 mg to 1,400 mg, more preferably from 10 mg to 100 mg, and unit dosage forms suitable for oral administration comprise from 2 mg to 1,400 mg of the compound admixed with a solid or liquid pharmaceutical carrier or diluent.

The compounds of formula I, enantiomers thereof, and pharmaceutically-acceptable salts thereof, may be used on their own or in the form of appropriate medicinal preparations for enteral or parenteral administration. According to a further aspect of the invention, there is provided a pharmaceutical composition including preferably less than 80% and more preferably less than 50% by weight of a compound of the invention in admixture with an inert pharmaceutically acceptable diluent or carrier.

Examples of diluents and carriers are:

- for tablets and dragees: lactose, starch, talc, stearic acid;
- capsules: tartaric acid or lactose;
- for injectable solutions: water, alcohols, glycerin, vegetable oils;
- for suppositories: natural or hardened oils or waxes.

There is also provided a process for the preparation of such a pharmaceutical composition, which comprises mixing the ingredients.

One aspect of the invention is the use of a compound according to the invention, an enantiomer thereof or a pharmaceutically acceptable salt thereof, in the manufacture of a medicament for the treatment or prophylaxis of one of the below mentioned diseases or conditions; and a method of treatment or prophylaxis of one of the above mentioned diseases or conditions, which comprises administering a therapeutically effective amount of a compound according to the invention, or an enantiomer thereof or a pharmaceutically acceptable salt thereof, to a patient.

Compounds to be used according to the invention are agonists of nicotinic acetylcholine receptors. While not

being limited by theory, it is believed that agonists of the $\alpha_7$ nAChR (nicotinic acetylcholine receptor) subtype should be useful in the treatment or prophylaxis of psychotic disorders and intellectual impairment disorders, and have advantages over compounds which are or are also agonists of the $\alpha_4$ nAChR subtype. Therefore, compounds which are selective for the $\alpha_7$ nAChR subtype are preferred. The use of compounds of the invention are indicated as pharmaceuticals, in particular in the treatment or prophylaxis of psychotic disorders and intellectual impairment disorders. Examples of psychotic disorders include schizophrenia, mania and manic depression, and anxiety. Examples of intellectual impairment disorders include Alzheimer's disease, learning deficit, cognition deficit, attention deficit, memory loss, and Attention Deficit Hyperactivity Disorder. The compounds of the invention may also be useful as analgesics in the treatment of pain (including chronic pain) and in the treatment or prophylaxis of Parkinson's disease, Huntington's disease, Tourette's syndrome, and neurodegenerative disorders in which there is loss of cholinergic synapses. The compounds may further be indicated for the treatment or prophylaxis of jetlag, for use in inducing the cessation of smoking, and for the treatment or prophylaxis of nicotine addiction (including that resulting from exposure to products containing nicotine).

It is also believed that compounds according to the invention are useful in the treatment and prophylaxis of ulcerative colitis.

## Pharmacology

The pharmacological activity of the compounds of the invention may be measured in the tests set out below:

### Test A - Assay for affinity at $\alpha_7$ nAChR subtype

$^{125}$I-$\alpha$-Bungarotoxin (BTX) binding to rat hippocampal membranes.

Rat hippocampi were homogenized in 20 volumes of cold homogenization buffer (HB: concentrations of constituents (mM): tris(hydroxymethyl)aminomethane 50; $MgCl_2$ 1; NaCl 120; KCl 5: pH 7.4). The homogenate was centrifuged for 5 minutes at 1000 xg, the supernatant was saved and the pellet re-extracted. The pooled supernatants were centrifuged for 20 minutes at 12000 xg, washed, and resuspended in HB. Membranes (30-80 $\mu$g) were incubated with 5 nM [$^{125}$I]$\alpha$-BTX, 1 mg/mL BSA (bovine serum albumin), test drug, and either 2 mM $CaCl_2$ or 0.5 mM EGTA [ethylene glycol-bis($\beta$-aminoethylether)] for 2 hours at 21 °C, and then filtered and washed 4 times over Whatman glass fibre filters (thickness C) using a Brandel cell harvester. Pretreating the filters for 3 hours with 1% (BSA/0.01% PEI (polyethyleneimine) in water was critical for low filter blanks (0.07% of total counts per minute). Nonspecific binding was described by 100 $\mu$M (-)-nicotine, and specific binding was typically 75%.

### Test B - Assay for affinity to the $\alpha_4$ nAChR subtype

[$^3$H]-(-)-nicotine binding.

Using a procedure modified from Martino-Barrows and Kellar (Mol Pharm (1987) 31:169-174), rat brain (cortex and hippocampus) was homogenized as in the [$^{125}$I]$\alpha$-BTX binding assay, centrifuged for 20 minutes at 12,000 xg, washed twice, and then resuspended in HB containing 100 $\mu$M diisopropyl fluorophosphate. After 20 minutes at 4 °C, membranes (approximately 0.5 mg) were incubated with 3 nM [$^3$H]-(-)-nicotine, test drug, 1 $\mu$M atropine, and either 2 mM $CaCl_2$ or 0.5 mM EGTA for 1 hour at 4 °C, and then filtered over Whatman glass fibre filters (thickness C) (pretreated for 1 hour with 0.5% PEI) using a Brandel cell harvester. Nonspecific binding was described by 100 $\mu$M carbachol, and specific binding was typically 84%.

### Binding data analysis for Tests A and B

$IC_{50}$ values and pseudo Hill coefficients (nH) were calculated using the non-linear curve fitting program ALLFIT (DeLean A, Munson P J and Rodbard D (1977) Am. J. Physiol., 235:E97-E102). Saturation curves were fitted to a one site model, using the non-linear regression program ENZFITTER (Leatherbarrow, R.J. (1987)), yielding KD values of 1.67 and 1.70 nM for the $^{125}$I-$\alpha$-BTX and [$^3$H]-(-)-nicotine ligands respectively. Ki values were estimated using the general Cheng-Prusoff equation:

$$K_i=[IC_{50}]/((2+([ligand]/[K_D])^n)^{1/n}-1)$$

where a value of n=1 was used whenever $^{n}H<1.5$ and a value of n=2 was used when $^{n}H \geq 1.5$. Samples were assayed in triplicate and were typically $\pm 5\%$. $K_i$ values were determined using 6 or more drug concentrations. The compounds of the invention are compounds with binding affinities ($K_i$) of less than 10 nM in either Test A or Test B, indicating that they are expected to have useful therapeutic activity.

The compounds of the invention have the advantage that they may be less toxic, be more efficacious, be longer acting, have a broader range of activity, be more potent, produce fewer side effects, are more easily absorbed or have other useful pharmacological properties.

## Claims

1. A compound of formula I:

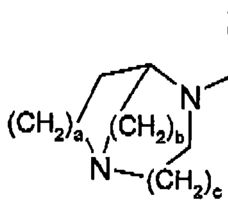

I

wherein:

a is 1, b is 2 and c is 1;
D is oxygen, and
R is selected from moieties of formulae II, III or IV:

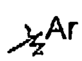

II

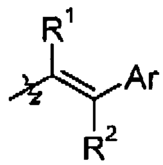

III

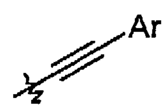

IV

wherein
$R^1$, and $R^2$ are independently selected from hydrogen, CN, $CF_3$, halogen, $C_{1-4}$alkyl,;
Ar is phenyl, naphthyl, benzofuranyl, benzo[b]thiophenyl, or 1H-indolyl;
wherein Ar may have have 0, 1 or more substituents independently selected from $CF_3$, halogen, $C_{1-4}$alkyl, or $OR^3$; where
$R^3$ is $C_{1-4}$alkyl;

or an enantiomer or pharmaceutically-acceptable salt thereof
with the proviso that said compound is not 4-benzoyl-1,4-diazabicyclo[3.2.1]octane.

2. A compound according to Claim 1, wherein Ar is a phenyl, furanyl or thiophenyl; or an enantiomer or pharmaceutically-acceptable salt thereof.

3. A compound according to Claim 1, wherein:

Ar is selected from phenyl, 2-furanyl or 3-furanyl, 2-thienyl or 3-thienyl, benzofuran-2-yl; benzofuran-3-yl, benzo [b]thiophen-2-yl or benzo[b]thiophen-3-yl;

or an enantiomer or pharmaceutically-acceptable salt thereof.

4. A compound according to Claim 1 selected from:

(1,4-diazabicyclo[3.2.2]non-4-yl)(phenyl)methanone;
(1,4-diazabicyclo[3.2,2]non-4-yl)(3-fluorophenyl)methanone;
(1,4-diazabicyclo[3.2.2]non-4-yl)(4-fluorophenyl)methanone;
(3-chlorophenyl)(1,4-diazabicyclo[3.2.2]non-4-yl)methanone;
(4-chlorophenyl)(1,4-diazabicyclo[3.2.2]non-4-yl)methanone;
(1,4-diazabicyclo[3.2.2]non-4-yl)(3,4-dichlorophenyl)methanone;
(3-bromophenyl)(1,4-diazabicyclo[3.2.2]non-4-yl)methanone;
(4-bromophenyl)(1,4-diazabicyclo[3,2,2]non-4-yl)methanone;
(1,4-diazabicyclo[3.2.2]non-4-yl)(3-iodophenyl)methanone;
(1,4-diazabicyclo[3.2.2]non-4-yl)(4-iodophenyl)methanone;
(1,4-diazabicyclo[3.2.2]non-4-yl)(4-trifluoromethylphenyl)methanone;
(1,4-diazabicyclo[3.2.2]non-4-yl)(4-methoxyphenyl)methanone;
(1,4-diazabicyclo[3.2.2]non-4-yl)(naphthalen-2-yl)methanone;
(1,4-diazabicyclo[3,2,2]non-4-yl)(benzofuran-2-yl)methanone;
(1,4-diazabicyclo[3.2.2]non-4-yl)(benzo[b]thiophen-2-yl)methanone;
1-(1,4-diazabicyclo[3.2.2]non4-yl)-3-phenylpropenone;
1-(1,4-diazabicyclo[3.2.2]non-4-yl)-3-phenylpropynone;
(E)-1-(1,4-diazabicyclo[3.2.2]non-4-yl)-3-(2-methoxyphenyl)-propenone;
(E)-1-(1,4-diazabicyclo[3.2.2]non-4-yl)-3-(2-methylphenyl)propenone;
(1,4-diaza-bicyclo[3.2.2]non-4-yl)-(1H-indol-5-yl)-methanone;
(1,4-diaza-bicyclo[3.2.2]non4-yl)-(methyl-1H-indol-2-yl)-methanone, and
(Z)-1-(1,4-diaza-bicyclo[3.2.2]non-4-yl)-2-fluoro-3-phenyl-propenone,

or an enantiomer or pharmaceutically-acceptable salt thereof.

**5.** A compound which is:

(1,4-diazabicyclo[3.2.2]non-4-yl)(4-trifluoromethoxyphenyl)methanone.

**6.** A compound according to any one of Claims 1 to 5, for use in therapy.

**7.** A compound according to any one of Claims 1 to 5, for use as a medicament.

**8.** Use of a compound as defined in any one of claims 1 to 5, in the manufacture of a medicament for the treatment or prophylaxis of psychotic disorders, intellectual impairment disorders, human diseases or conditions in which activation of the α7 nicotinic receptor is beneficial, Alzheimer's disease, learning deficit, cognition deficit, attention deficit, memory loss, Lewy Body Dementia, Attention Deficit Hyperactivity Disorder, anxiety, schizophrenia, mania or manic depression, Parkinson's disease, Huntington's disease, Tourette's syndrome, neurodegenerative disorders in which there is loss of cholinergic synapse, jetlag, cessation of smoking, nicotine addiction including that resulting from exposure to products containing nicotine, pain, ulcerative colitis or irritable bowel syndrome.

**9.** A pharmaceutical composition comprising a compound of formula I, as defined in any one of claims 1 to 5, together with at least one pharmaceutically-acceptable excipient or diluent.

## Patentansprüche

**1.** Verbindung der Formel I:

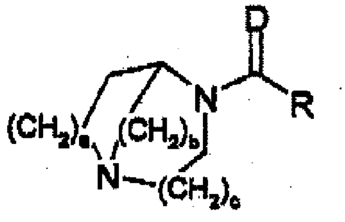

I

worin:

a für 1 steht, b für 2 steht und c für 1 steht;
D für Sauerstoff steht und
R unter Gruppierungen der Formeln II, III oder IV ausgewählt ist:

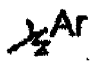
II

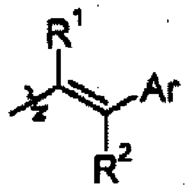
III

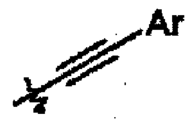
IV

worin
$R^1$ und $R^2$ unabhängig voneinander unter Wasserstoff, CN, $CF_3$, Halogen oder $C_{1-4}$-Alkyl ausgewählt sind;
Ar für Phenyl, Naphthyl, Benzofuranyl, Benzo[b]-thiophenyl oder 1H-Indolyl steht;
wobei Ar 0, 1 oder mehr unabhängig voneinander unter $CF_3$, Halogen, $C_{1-4}$-Alkyl oder $OR^3$ ausgewählte Substituenten aufweisen kann; wobei
$R^3$ für $C_{1-4}$-Alkyl steht;
oder ein Enantiomer oder pharmazeutisch annehmbares Salz davon
mit der Maßgabe, daß es sich bei der Verbindung nicht um 4-Benzoyl-1,4-diazabicyclo[3.2.1]octan handelt.

**2.** Verbindung nach Anspruch 1, worin Ar für Phenyl, Furanyl oder Thiophenyl steht; oder ein Enantiomer oder pharmazeutisch annehmbares Salz davon.

**3.** Verbindung nach Anpruch 1, worin:

Ar unter Phenyl, 2-Furanyl oder 3-Furanyl, 2-Thienyl oder 3-Thienyl, Benzofuran-2-yl, Benzofuran-3-yl, Benzo [b]thiophen-2-yl oder Benzo[b]-thiophen-3-yl ausgewählt ist; oder ein Enantiomer oder pharmazeutisch annehmbares Salz davon.

**4.** Verbindung nach Anpruch 1, ausgewählt unter:

(1,4-Diazabicyclo[3.2.2]non-4-yl) (phenyl)methanon;
(1,4-Diazabicyclo[3.2.2]non-4-yl) (3-fluorphenyl)-methanon;
(1,4-Diazabicyclo[3.2.2]non-4-yl) (4-fluorphenyl)-methanon;
(3-Chlorphenyl)(1,4-diazabicyclo[3.2.2]non-4-yl)-methanon;
(4-Chlorphenyl)(1,4-diazabicyclo[3.2.2]non-4-yl)-methanon;
(1,4-Diazabicyclo[3.2.2]non-4-yl)(3,4-dichlorphenyl)methanon;
(3-Bromphenyl)(1,4-diazabicyclo[3.2.2]non-4-yl)-methanon;
(4-Bromphenyl)(1,4-diazabicyclo[3.2.2]non-4-yl)-methanon;
(1,4-Diazabicyclo[3.2.2]non-4-yl) (3-iodphenyl)-methanon;
(1,4-Diazabicyclo[3.2.2]non-4-yl) (4-iodphenyl)-methanon;
(1,4-Diazabicyclo[3.2.2]non-4-yl) (4-trifluormethylphenyl)methanon;
(1,4-Diazabicyclo[3.2.2]non-4-yl) (4-methoxyphenyl)methanon;
(1,4-Diazabicyclo[3.2.2]non-4-yl) (naphthalin-2-yl)methanon;
(1,4-Diazabicyclo[3.2.2]non-4-yl) (benzofuran-2-yl)methanon;
(1,4-Diazabicyclo[3.2.2]non-4-yl) (benzo[b]-thiophen-2-yl)methanon;
1-(1,4-Diazabicyclo[3.2.2]non-4-yl)-3-phenylpropenon;
1-(1,4-Diazabicyclo[3.2.2]non-4-yl)-3-phenylpropinon;
(E)-1-(1,4-Diazabicyclo[3.2.2]non-4-yl)-3-(2-methoxyphenyl)propenon;
(E)-1-(1,4-Diazabicyclo[3.2.2]non-4-yl)-3-(2-methylphenyl)propenon;
(1,4-Diazabicyclo[3.2.2]non-4-yl) (1H-indol-5-yl)-methanon;

(1,4-Diazabicyclo[3.2.2]non-4-yl) (methyl-1H-indol-5-yl)methanon und
(2)-1-(1,4-Diazabicyclo[3.2.2]non-4-yl)-2-fluor-3-phenylpropanon;

oder ein Enantiomer oder pharmazeutisch annehmbares Salz davon.

**5.** Verbindung, bei der es sich um (1,4-Diazabicyclo[3.2.2]non-4-yl) (4-trifluormethoxyphenyl)methanon handelt.

**6.** Verbindung nach einem der Ansprüche 1 bis 5 zur Verwendung bei der Therapie.

**7.** Verbindung nach einem der Ansprüche 1 bis 5 zur Verwendung als Arzneimittel.

**8.** Verwendung einer Verbindung nach einem der Ansprüche 1 bis 5 bei der Herstellung eines Arzneimittels zur Behandlung oder Prophylaxe von psychotischen Störungen, Intelligenzstörungen, Krankheiten oder Zuständen des Menschen, bei denen die Aktivierung des nicotinischen Rezeptors $\alpha7$ vorteilhaft ist, Alzheimer-Krankheit, Lernschwäche, Denkschwäche, Konzentrationsstörungen, Gedächtnisschwund, Lewy-Körperchen-Demenz, hyperkinetischem Syndrom, Angst, Schizophrenie, Manie oder manischer Depression, Parkinson-Krankheit, Chorea Huntington, Tourette-Syndrom, neurodegenerativen Erkrankungen mit Verlust cholinerger Synapsen, Jet-lag, Raucherentwöhnung, Nicotinabhängigkeit einschließlich der sich aus der Exposition gegenüber nicotinhaltigen Produkten ergebenden Abhängigkeit, Schmerzen, Colitis ulcerosa oder Reizkolon.

**9.** Pharmazeutische Zusammensetzung, enthaltend eine Verbindung der Formel I nach einem der Ansprüche 1 bis 5 zusammen mit mindestens einem pharmazeutisch annehmbaren Hilfsstoff oder Verdünnungsmittel.

## Revendications

**1.** Composé de formule I :

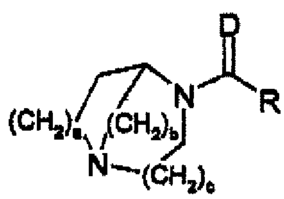

I

dans laquelle :

a est 1, b est 2 et c est 1 ;
D est oxygène ; et
R est choisi parmi les motifs de formules II, III ou IV :

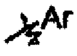

II

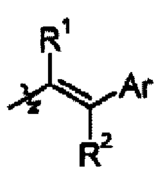

III

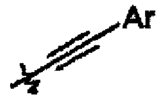

IV

dans lesquelles :
$R^1$ et $R^2$ sont choisis, indépendamment, parmi hydrogène, CN, $CF_3$, halogène, $C_{1-4}$alkyle ;
Ar est phényle, naphtyle, benzofuranyle, benzo-[b]thiophényle ou 1H-indolyle ;
où Ar peut avoir 0, 1 ou plusieurs substituants choisis, indépendamment, parmi $CF_3$, halogène, $C_{1-4}$alkyle or

$OR^3$ ; où
$R^3$ est $C_{1-4}$alkyle ;

ou un énantiomère ou un sel pharmaceutiquement acceptable de celui-ci ;
à condition que ledit composé ne soit pas le 4-benzoyl-1,4-diazabicyclo[3,2,1]octane.

2. Composé selon la revendication 1, **caractérisé en ce que** Ar est un phényle, furanyle ou thiophényle ; ou un énantiomère ou un sel pharmaceutiquement acceptable de celui-ci.

3. Composé selon la revendication 1, **caractérisé en ce que :**

Ar est choisi parmi phényle, 2-furanyle ou 3-furanyle, 2-thiényle ou 3-thiényle, benzofuran-2-yle, benzofuran-3-yle, benzo[b]thiophén-2-yle ou benzo[b]thiophén-3-yle ;
ou un énantiomère ou un sel pharmaceutiquement acceptable de celui-ci.

4. Composé selon la revendication 1 choisi parmi :

la (1,4-diazabicyclo[3,2,2]non-4-yl)(phényl)-méthanone ;
la (1,4-diazabicyclo[3,2,2]non-4-yl)(3-fluorophényl)méthanone ;
la (1,4-diazabicyclo[3,2,2]non-4-yl)(4-fluorophényl)méthanone ;
la (3-chlorophényl)(1,4-diazabicyclo[3,2,2]non-4-yl)méthanone ;
la (4-chlorophényl)(1,4-diazabicyclo[3,2,2]non-4-yl)méthanone ;
la (1,4-diazabicyclo[3,2,2]non-4-yl) (3,4-dichlorophényl)méthanone ;
la (3-bromophényl)(1,4-diazabicyclo[3,2,2]non-4-yl)-méthanone ;
la (4-bromophényl)(1,4-diazabicyclo[3,2,2]non-4-yl)-méthanone ;
la (1,4-diazabicyclo[3,2,2]non-4-yl) (3-iodophényl)-méthanone ;
la (1,4-diazabicyclo[3,2,2]non-4-yl) (4-iodophényl)-méthanone ;
la (1,4-diazabicyclo[3,2,2]non-4-yl) (4-trifluorométhylphényl)méthanone ;
la (1,4-diazabicyclo[3,2,2]non-4-yl) (4-méthoxyphényl)méthanone ;
la (1,4-diazabicyclo[3,2,2]non-4-yl) (naphtalén-2-yl)méthanone ;
la (1,4-diazabicyclo[3,2,2]non-4-yl) (benzofuran-2-yl)méthanone ;
la (1,4-diazabicyclo[3,2,2]non-4-yl) (benzo[b]thiophén-2-yl)méthanone ;
la 1-(1,4-diazabicyclo[3,2,2]non-4-yl)-3-phénylpropénone ;
la 1-(1,4-diazabicyclo[3,2,2]non-4-yl)-3-phénylpropynone ;
la (E)-1-(1,4-diazabicyclo[3,2,2]non-4-yl)-3-(2-méthoxyphényl)propénone ;
la (E)-1-(1,4-diazabicyclo[3,2,2]non-4-yl)-3-(2-méthylphényl)propénone ;
la (1,4-diazabicyclo[3,2,2]non-4-yl)(1H-indol-5-yl)-méthanone ;
la (1,4-diazabicyclo[3,2,2]non-4-yl) (méthyl-1H-indol-2-yl)méthanone ; et
la (Z)-1-(1,4-diazabicyclo[3,2,2]non-4-yl)-2-fluoro-3-phénylpropénone ;

ou un énantiomère ou un sel pharmaceutiquement acceptable de celui-ci.

5. Composé qui est :

la (1,4-diazabicyclo[3,2,2]non-4-yl)(4-trifluorométhoxyphényl)méthanone.

6. Composé selon l'une quelconque des revendications 1 à 5, destiné à être utilisé en thérapie.

7. Composé selon l'une quelconque des revendications 1 à 5, destiné à être utilisé comme médicament.

8. Utilisation d'un composé, tel que défini dans l'une quelconque des revendications 1 à 5, dans la fabrication d'un médicament destiné au traitement ou à la prophylaxie de troubles psychotiques, de troubles de déficience intellectuelle, de maladies ou d'affections humaines dans lesquelles l'activation du récepteur nicotinique $\alpha 7$ est bénéfique, de la maladie d'Alzheimer, de la déficience de l'apprentissage, de la déficience cognitive, du déficit de l'attention, de la perte de mémoire, de la démence à corps de Lewy, du trouble de l'attention et de l'hyperactivité, de l'anxiété, de la schizophrénie, de la manie ou de la psychose maniaco-dépressive, de la maladie de Parkinson, de la maladie de Huntington, du syndrome de Tourette, de troubles neurodégénératifs dans lesquels il y a perte de la synapse cholinergique, du syndrome du décalage horaire, du sevrage tabagique, de la dépendance à la nicotine - y compris

celle qui résulte de l'exposition aux produits contenant de la nicotine - de la douleur, de la colite ulcéreuse ou du syndrome de l'intestin irritable.

**9.** Composition pharmaceutique comprenant un composé de formule I, tel que défini dans l'une quelconque des revendications 1 à 5, conjointement avec au moins un excipient ou diluant pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0058311 A


### Non-patent literature cited in the description


- Nicotinic Acetylcholine Receptors: Molecular Biology, Chemistry and Pharmacology. **MCDONALD et al.** Annual Reports in Medicinal Chemistry. Academic Press Inc, 1995, vol. 30, 41-50
- **WILLIAM et al.** Neuronal Nicotinic Acetylcholine Receptors. *Drug News & Perspectives,* 1994, vol. 7, 205-223
- **MARTINO-BARROWS ; KELLAR.** *Mol Pharm,* 1987, vol. 31, 169-174
- **DELEAN A ; MUNSON P J ; RODBARD D.** *Am. J. Physiol.,* 1977, vol. 235, E97-E102